# EUROPEAN PATENT SPECIFICATION

(11) **EP 3 986 507 B1**
(45) Date of publication and mention of the grant of the patent: **19.11.2025**
(21) Application number: 20745275.6
(22) Date of filing: 22.06.2020
(51) Int. Cl.: A61M 5/20, A61M 5/32, A61M 5/50, A61M 5/24, A61M 5/28

(54) **MEDICAL INJECTION SYSTEM**
MEDIZINISCHES INJEKTIONSSYSTEM
SYSTÈME D'INJECTION

(30) Priority: 21.06.2019 US 201962864572 P
(43) Date of publication of application: 27.04.2022
(73) Proprietor: Preci Health SA, 2000 Neuchâtel (CH)
(72) Inventor: MARGAIRAZ, Philippe, 2300 La Chaux-de-Fonds (CH); DA SILVA, Carlos, 2114 Fleurier (CH); MAFFLI, Luc, 1700 Fribourg (CH); JACCARD, Alain, 1450 Ste-Croix (CH); VOUILLAMOZ, Lucien, 8835 Feusisberg (CH)
(74) Representative: Mötteli-Mantelli, Novella
(86) International application number: PCT/IB2020/055874
(87) International publication number: WO 2020/255105

(56) References cited:
- EP-A1- 0 180 628
- EP-B1- 0 180 628
- WO-A2-2008/064092
- US-A1- 2013 317 427
- US-A1- 2018 133 395

## Description

### Background of the Invention

Known fluid dispensing devices for medical uses are disclosed, for example, in document EP0180628 that relates an injection syringe adapted for mixing a plurality of liquids during the injection process, and in document WO 2008/064092 that relates to an apparatus and a method for automatic medicament injection comprising an actuator able to move the element responsible for the injection through one or more springs or pressurized cylinders.

A previous version of a fluid dispensing device is also mentioned in document US2018/133395 by the Applicant, wherein two concentric springs are used to move the element responsible for the injection.

### Summary of the Invention

This invention relates to devices for injecting fluids, optionally including medicine, into a living organism in a controlled and automated manner.

In particular the present invention refers to a fluid dispensing device adapted for intramuscular and/or subcutaneous injection into a living organism according to claim 1. Preferred embodiments of the invention are defined in the appended dependent claims.

The fluid dispensing device is adapted to dispense a measured amount of fluid, preferably into the muscle, typically intramuscular, of a living organism is provided. The fluid dispensing device is also adapted to dispense a measured amount of fluid intracutaneous or subcutaneous to a living organism. The device has an energy storage arrangement providing energy in at least two bursts. The first burst is provided from a first energy source and is that which is necessary to pierce the skin of the living organism and insert the needle to the injection site. The second burst is provided by a second energy source and is that which is necessary to pump the fluid out of the reservoir and into the living organism and to retract the needle out of the living organism. Together with a prescribed operating sequence of the device, use of the two sources of energy contained in the device is optimized to provide the at least two bursts of energy.

The fluid dispensing device has a main housing enclosing the operative components of the device, a fluid reservoir located within the main housing, an injection assembly including a needle, and a trigger mechanism. The fluid reservoir mentioned in the instant description is typically a cartridge, although other reservoirs can be used. The needle has a first end and a second end and is adapted for interfacing, on the first end, with a septum disposed in the reservoir containing the fluid to be dispensed, and at a second end thereof, for inserting into a living organism. The mechanism contains the energy necessary to insert the needle into the living organism, dispense the fluid, and retract the needle, and ensures the completion of an operational cycle without any other action from the user other than triggering (which is typically accomplished while holding the device firmly in place). The first and second ends of the needle pierce respective surfaces while translating together in a direction parallel to the skin for the first end of the needle and substantially non orthogonal to the surface of the living oganism's skin for the second end of the needle. Once the septum is pierced, the needle is in fluid communication with the fluid reservoir and the device expels the fluid through the needle into the living organism.

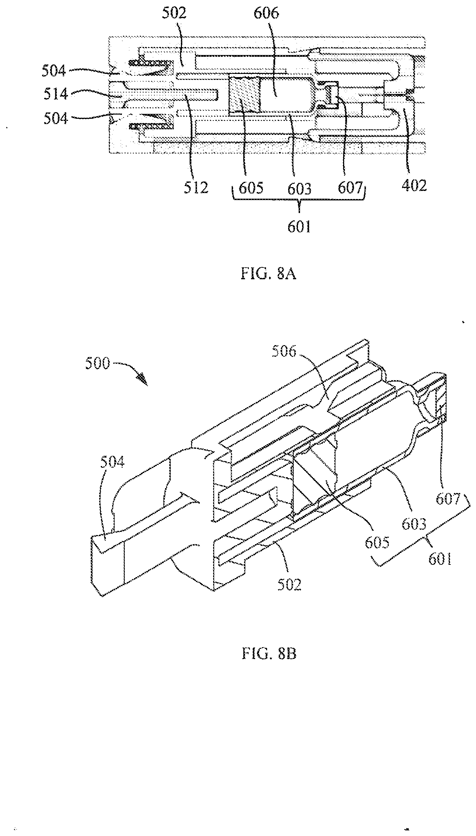

The invention is disclosed herein in three principal embodiments. The second and third embodiments are alternative embodiments of the first embodiment. Particularities of the second embodiment are shown in FIGs. **8A, 8B****,** **10A, 10B** and **12****.** Otherwise, if, for certain operational steps, no particular figure of the second embodiment specifically discloses this feature or step, the corresponding figure of the first embodiment is representative of all embodiments to support this feature or step in the other embodiments. The first and the second embodiment differ mainly in their mechanical proportions. The first embodiment is intended for intramuscular application, i.e. performing an injection typically of 2-3cm into the patient's soft tissue, while the second embodiment is intended for subcutaneous application, i.e. performing an injection typically 1-2mm into the patient's soft tissue. Hence, the first embodiment typically includes a long needle, the long needle having a length adapted to penetrate 2-3cm into the patient's tissue, while the second embodiment typically includes a short needle, the short needle having a length adapted to penetrate 1-2mm into the patient's soft tissue. Each of the first and the second embodiment includes a cartridge. The cartridge of the first embodiment (containing about 1 ml of fluid) is adapted to contain concentrated drugs, while the cartridge of the second embodiment (containing about 3 ml of fluid) is adapted to contain drugs with standard concentration.

In a third embodiment, the fluid dispensing device is retained (via the user, an elastic band or an adhesive or adhesive pad) against the skin of the living organism and may be manually, automatically or remotely actuated to inject a fluid into the living organism. Such features of the third embodiment may readily be integrated in the first and second embodiments.

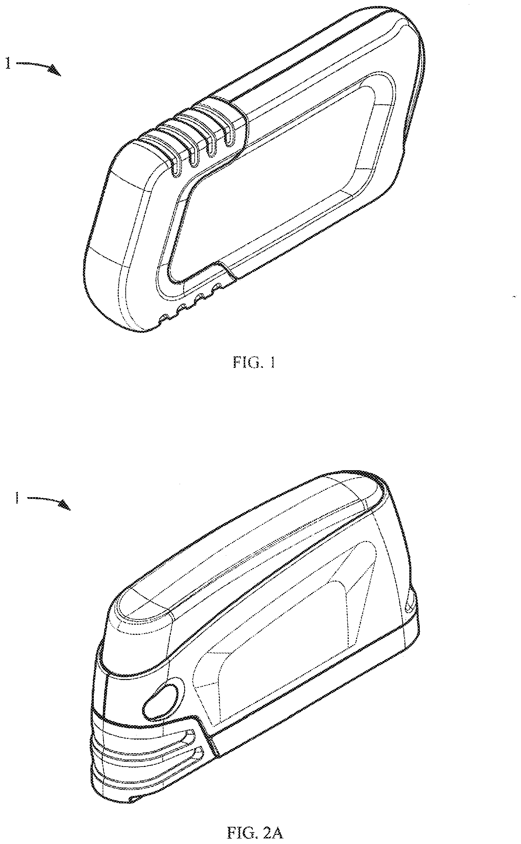

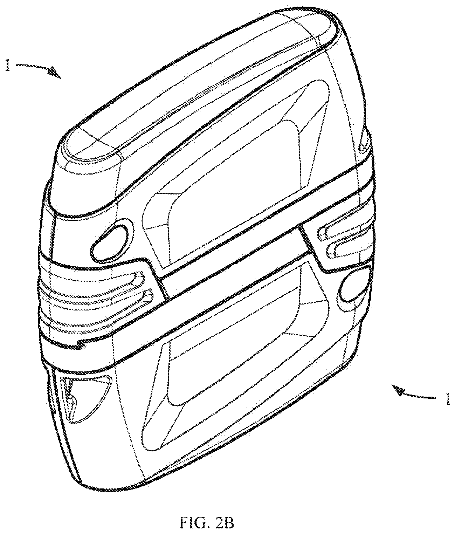

In a fourth embodiment, devices are stacked (see **FIG. 2****).** Such feature of the fourth embodiment may also readily be integrated in the first three embodiments.

In a fifth embodiment, instead of a replaceable cartridge, the reservoir can be filled with a fluid into a dedicated reservoir in the device itself, such feature also easily being integrated into the other embodiments described herein.

It is an object of the invention to provide an easy to use, user friendly device that allows any person, even the patients themselves, to perform an injection of a fluid, of particular interest, a COVID-19 vaccine, without the intervention of medical personell, thereby reducing the burden on world healthcare systems.

It is another object of the invention to provide a user friendly device that allows a patient to inject himself without requiring the user to make an aggressive gesture like stabing himself.

It is another object of the invention to provide a device that can step up to the challenge of new molecular therapeutic innovations such as high viscosity fluids whose characteristics otherwise limit administration options.

In an advantage, the embodiment of the invention using more than one needle enables simultaneous injection of several smaller doses of fluid into a living organism instead of one larger dose, generating less local tissue damage and therefore reducing the pain of the injection for the patient.

It is another object of the invention to provide a device with and extended product life cycle.

It is another object of the invention to provide a device that retracts the needle to prevent inadvertent harm that might be caused by an exposed needle.

It is another object of the invention to provide the option of injecting at an angle as the needle passes through a mandrel.

It is another object of the invention to prevent unsanitary or perhaps inadvertent reuse of the device, thus providing a safer means of injecting a fluid.

### Brief Description of the Drawings

**FIG. 1** is a perspective view of a first embodiment of a device according to the invention.
**FIG. 2A** is a perspective view of a second embodiment of a device according to the invention.
**FIG. 2B** is a view of two devices according to the second embodiment of the invention attached together.
**FIG. 3** is a partially exploded perspective view of the first embodiment of the invention while being prepared for use.
**FIG. 4** is a partially exploded perspective view of the second embodiment of the invention while being prepared for use.
**FIG. 5A** is a partial cross-sectional view of a mechanism according to the invention.
**FIG. 5B** is a cross-sectional view of the mechanism of the invention.
**FIG. 6A** is a partial perspective view of the needle subassembly according to the invention.
**FIG. 6B** is a partial perspective view of the insertion subassembly and the needle subassembly according to the invention.
**FIG. 6C** is a partial perspective view of the subassemblies of **FIG. 6B****,** from a first rearward perspective.
**FIG. 6D** is a partial perspective view of the subassemblies of **FIG. 6B****,** from below.
**FIG. 7A** is a perspective view of the injection subassembly according to the invention.
**FIG. 7B** is a cross-sectional view of the injection subassembly of **FIG. 7A** showing the internal reservoir subassembly according to the invention.
**FIG. 8A** is a cross-sectional view of an alternative example of the injection subassembly as shown in **FIGs. 6A** and **6B** not belonging to the invention as defined in the claims but useful for the understanding thereof.
**FIG. 8B** is another cross-sectional view of the alternative example of **FIG. 8A** not belonging to the invention as defined in the claims but useful for the understanding thereof.
**FIG. 9A** is a cross-section view of the mechanism of the invention at the step 6 of its use.
**FIG. 9B to 9D** are cross-sectional views of the mechanism of the invention at the step 7 of its use.
**FIGs. 10A-10B** are cross-sectional views of the mechanism of an alternative example at step 7 of its use, not belonging to the invention as defined in the claims but useful for the understanding thereof.
**FIG. 11** is a cross-sectional view of the mechanism of the invention at the step 8 of its use.
**FIG. 12** is a cross-sectional view of the mechanism of an alternative example at the step 8 of its use, not belonging to the invention as defined in the claims but useful for the understanding thereof.
**FIG. 13** is a cross-sectional view of the mechanism of the invention at the step 9 of its use.
**FIGs. 14A-14B** are cross-sectional views of the mechanism of the invention at the step 10 of its use.
**FIG. 15** is a side view of the needle.
**FIG 16** is a cross-sectional view of an alternative embodiment.

Those skilled in the art will appreciate that elements in the Figures are illustrated for simplicity and clarity and have not necessarily been drawn to scale. For example, dimensions may be exaggerated relative to other elements to help improve understanding of the invention and its embodiments. Furthermore, when the terms 'first', 'second', and the like are used herein, their use is intended for distinguishing between similar elements and not necessarily for describing a sequential or chronological order. Moreover, relative terms like 'front', 'back', 'top' and 'bottom', and the like in the Description and/or in the claims are not necessarily used for describing exclusive relative position. Those skilled in the art will therefore understand that such terms may be interchangeable with other terms, and that the embodiments described herein are capable of operating in other orientations than those explicitly illustrated or otherwise described.

### Detailed Description of the Preferred Embodiment

The following description illustrates the best mode of the invention known the inventors as of the filing date hereof.

Referring now to **FIGs. 1, 2A** and **2B****,** the device 1 of the invention has a narrow profile shape that is easy to carry in a purse or a pocket. Optionally, a pair of devices may be provided attached together for convenience .

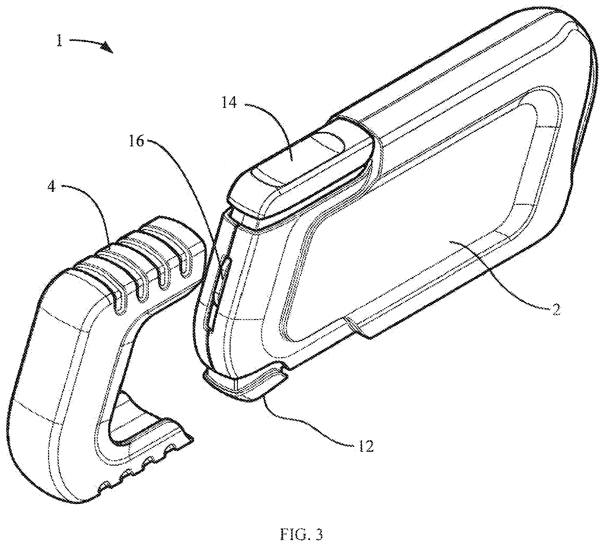

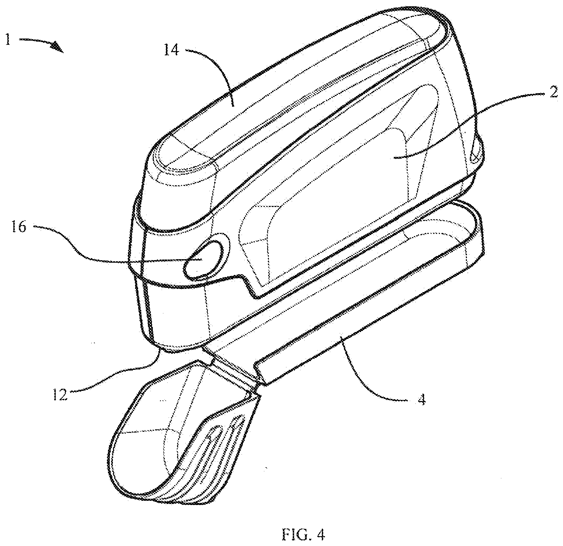

Referring now to **FIGs. 3** and **4****,** the device 1 has a main body 2 and a cover 4 that the user removes in order to have access to the push button 14. An indicator 16 allows the user to check whether the device has already been used or is available for use. Once the cover 4 has been removed, the user applies the device main body 2 against the skin of a living organism. An optional pressure-sensitive safety release mechanism 12 releases the push button 14 only when the pressure applied on the skin of the living organism is above a predefined threshold to ensure correct disposition/retention for optimal operation of the device prior to allowing the push button 14 to be pressed for triggering.

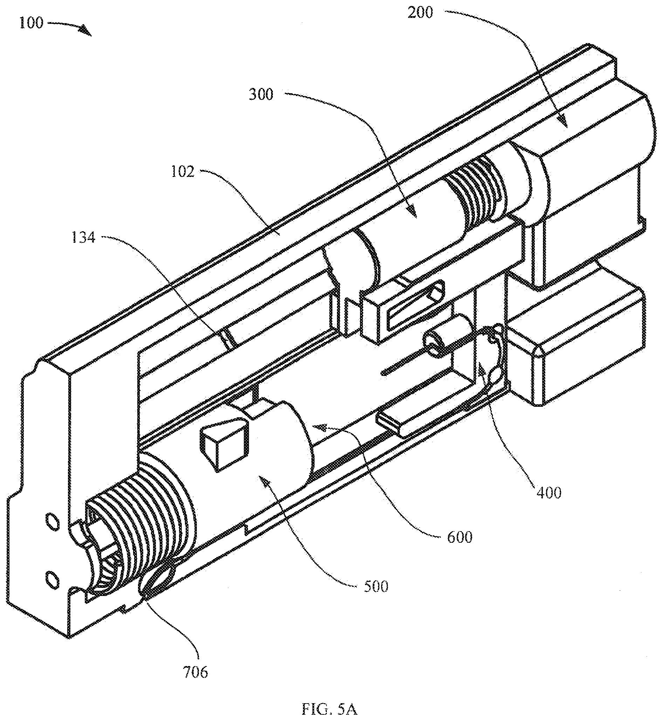

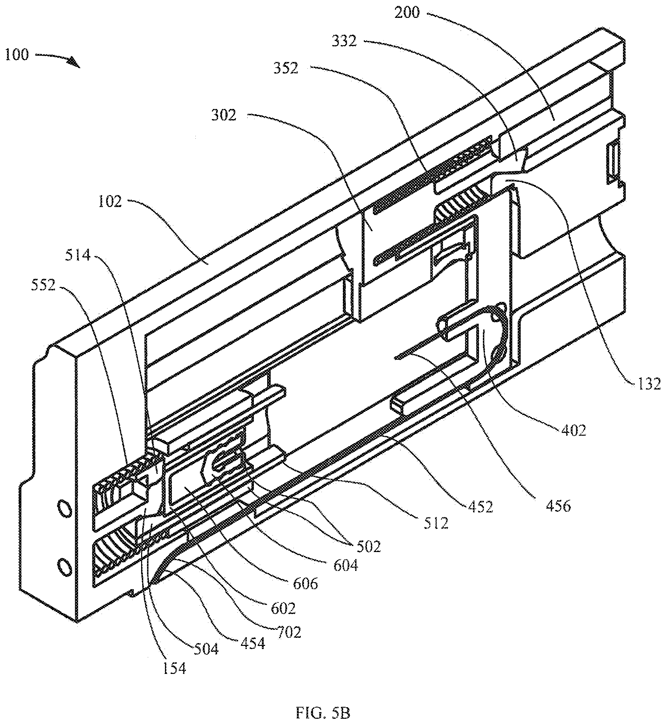

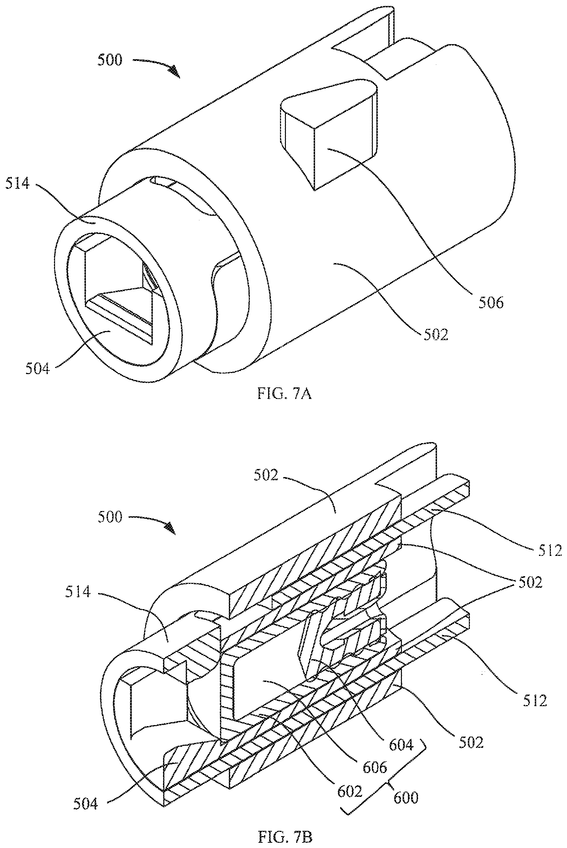

Referring now to **FIGs. 5A** and **5B****,** the mechanism 100 of the medical device 1 of the invention is made up of five main subassemblies which are contained in the main structure 102: the trigger mechanism 200, the insertion subassembly 300, the needle subassembly 400, the dispensing subassembly 500 and the reservoir subassembly 600 (shown in **FIG. 7B****).** The main structure may be made of injected polymer, or any other suitable material.

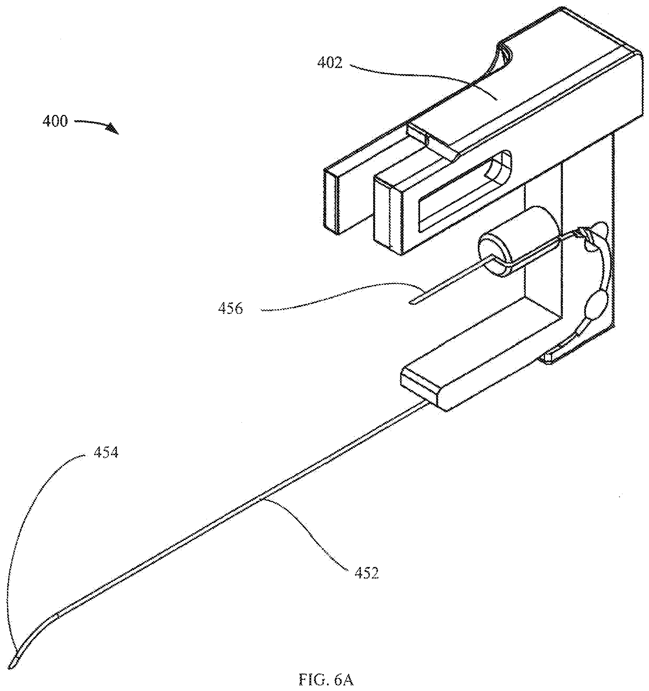

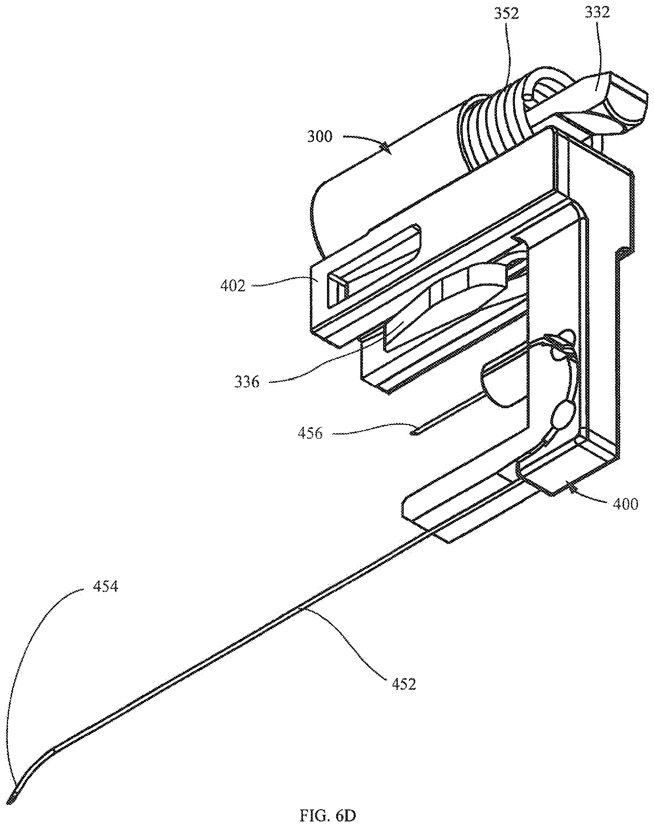

Referring now to **FIG. 6A****,** the needle subassembly 400 is made up of a structure 402, which may be made of injected polymer, or any other suitable material. The needle subassembly structure 402 holds the needle 452, which has an injection end 454 through which fluid is injected into a living organism, and an extraction end 456 into which the fluid is extracted from the reservoir. The structure 402 has the appropriate shape to be guided and oriented in the mechanism 100 and to provide the latch and locking features for operation of the mechanism 100. The needle 452 is made of stainless steel, or any other suitable material.

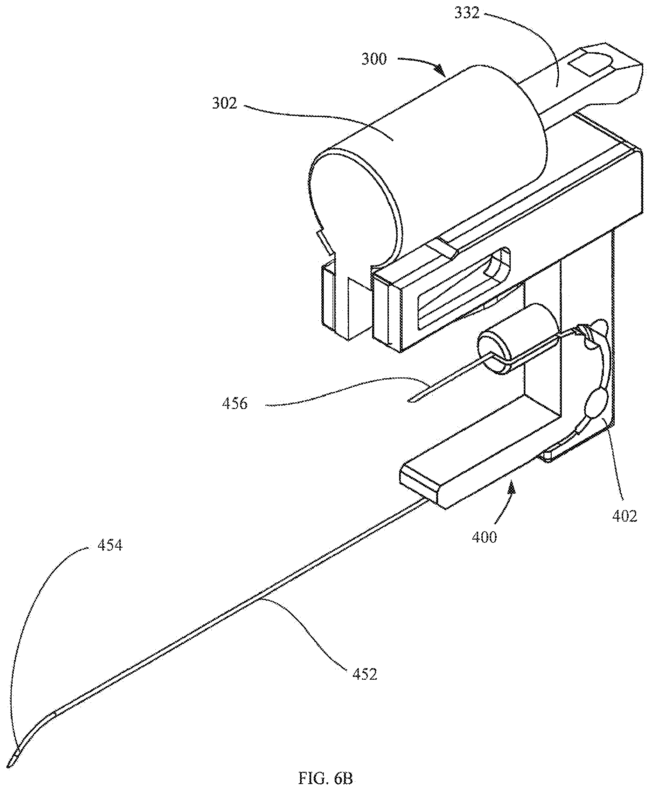

Referring now to **FIGs. 6B to 6D****,** the insertion subassembly 300 is attached to the needle subassembly 400 prior to beginning the operational cycle of the mechanism 100. The insertion subassembly 300 is made up of a structure 302, which may be made of injected polymer, or any other suitable material. The structure 302 contains the insertion spring 352, which is compressed prior to device operation (e.g., at assembly). The spring may be made of stainless steel or any other suitable material. The structure 302 has the appropriate shape to be guided in the mechanism 100 and to provide the latch and locking features 332, 334 for operation of the device. The structure also includes a release feature 336 for releasing the needle subassembly 400 from the insertion subassembly 300 at step 10 of the operation of the mechanism 100.

Referring now to **FIGs. 7A** and **7B****,** the dispensing assembly 500 contains the reservoir subassembly 600. The dispensing assembly 500 is made up of a structure 502 and an intermediate part 512, which may be made of injected polymer, or any other suitable material. The structure 502 and the intermediate part 512 have the appropriate shape for guiding the mechanism 100 relative to adjacent components, and to provide the latch, locking and triggering features 504, 506, 514 for operation of the mechanism 100. The reservoir subassembly 600 is made up of a container 602, a septum 604, and contains the fluid 606 intended to be injected in the living organism by the operation of the device 1. The reservoir container 602 may be made of glass, or any other suitable material. The septum 604 is made of rubber-like, elastic, self-sealing material, or any other suitable material, and includes the necessary features so as to be able to be pierced by the needle extraction end 456 and to sealingly slide into the container 602 to act as a piston as required at the step 7 of the operation of the mechanism 100. Features integrated in the septum 604 to ensure the intended behavior may include a rigid or semi-rigid structure or a composite of such structures.

Referring now to **FIGs. 8A to 8B****,** in an alternative example, instead of the custom reservoir 600, the device contains a standard cartridge 601 as are readily available on the market. The cartridge 601 is made up of a container 603, a stopper 605 which acts as a piston to fill the cartridge on the fill & finish lines (not represented), and a septum 607. The cartridge 601 contains the fluid 606 to be injected into the living organism. At step 7, the stopper 605 is to be used as a piston to expel the fluid 606 to be injected, while the septum 607 is pierced by the needle extraction end 456.

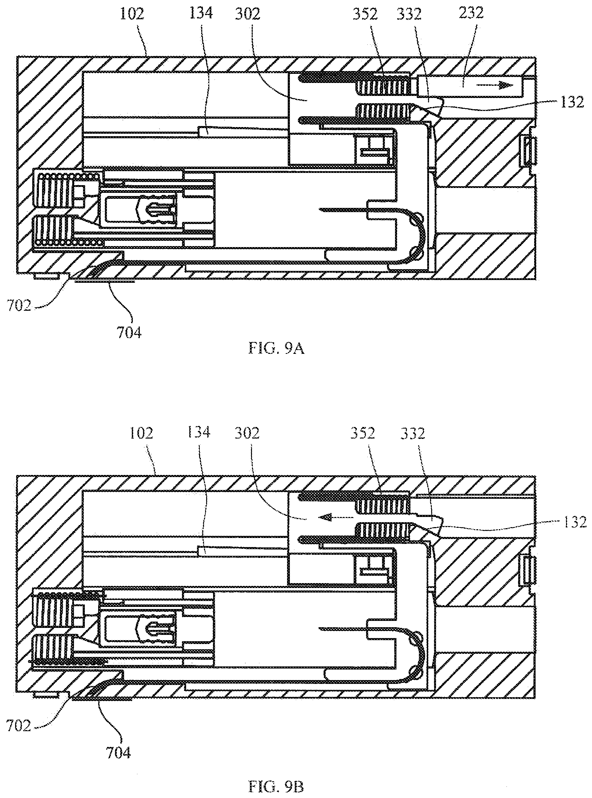

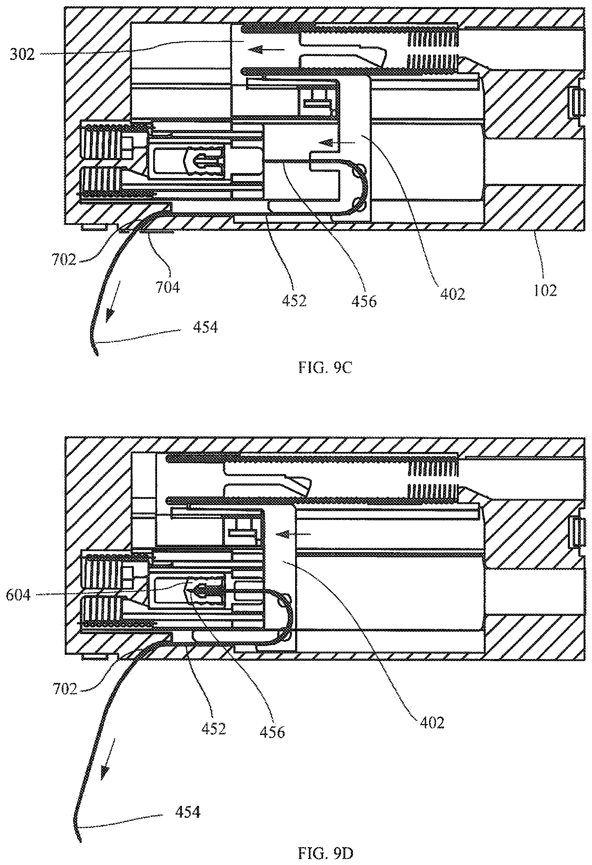

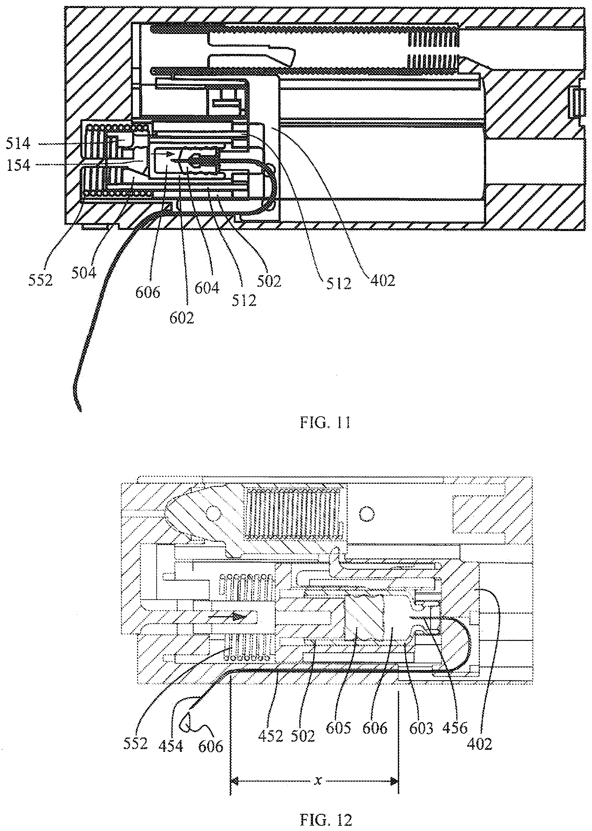

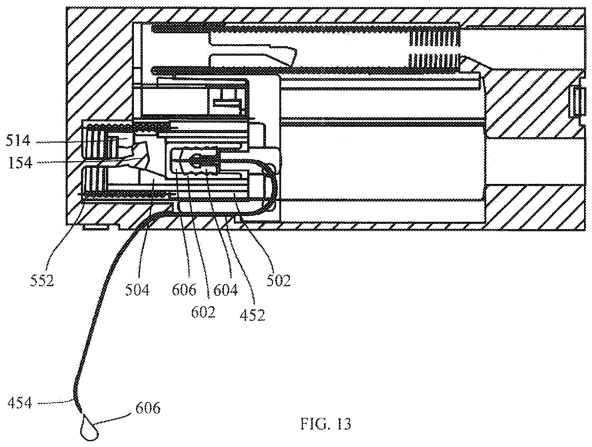

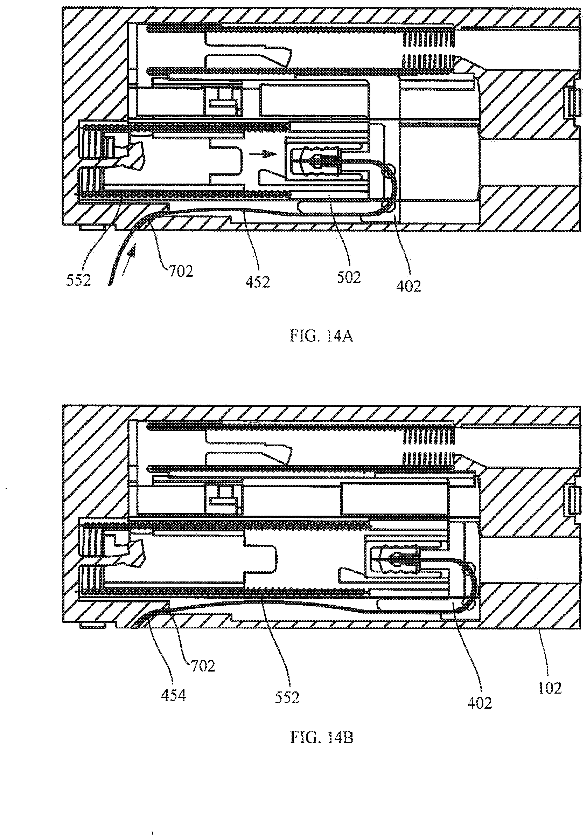

The sequence of operation of the device is as follows:
1. In case the device 1 is attached to a second device 1 as a pair (see FIG. 2), the user detaches the devices 1 in order to be able to use one device at a time.
2. The user removes the cover 4 (FIGs. **3** and **4****).**
3. The user applies the device body 2 against the skin of the living organism.
4. The pressure applied against the skin of the living organism is increased until a stable juxtaposition against the skin is attained for injection or until sufficient so that an optional detector 12 releases the push button 14 (FIGs. **3** and **4** - at some predefined level of pressure). Alternatively the optional detector 12 actuates the trigger mechanism 200, in which case, we jump to step 6.
5. The user pushes the push button 14, actuating the trigger mechanism 200.
6. The trigger mechanism 200 releases the insertion subassembly 300 (FIG. **9A****).**
7. The insertion subassembly 300 pushes the needle assembly 400, until the needle injecting end 454 is able to insert into the living organism to an injection depth, and the needle extraction end 456 has pierced the reservoir septum 604 (FIGs. **9B** to **9D****).**
8. Upon reaching this position, the insertion subassembly 300 is locked to the main structure 102 by a latch system 334, 134. As a consequence, the needle subassembly 400, which is still attached to the insertion subassembly 300, is also locked in place. At the same time, the dispensing assembly 500 is released, pushing the fluid reservoir subassembly 600 against the needle subassembly 400 (FIGs. **11** and **12****).**
9. The septum 604 is displaced towards the bottom of the fluid reservoir 602, acting as a piston and dispensing the fluid 606 through the needle extraction end 456, through the needle 452, through the needle injection end 454 into the living organism (FIG. **13****).**
10. Optionally, after the dispensing subassembly 500 has emptied the reservoir, the needle subassembly 400 is released from the insertion subassembly 300, and is pushed back to its start position, retracting the needle 452 until the needle injection end 454 is fully retracted within the main structure 102 (FIGs. **14A** and **14B****).**

The combination of the herein described device and operating sequence brings the key advantage of optimizing the use of 2 sources of energy contained in the device (2 springs) using their maximum energy (when the springs are compressed at their maximum) to provide 2 bursts of energy: the first burst that is necessary to pierce the skin of the living organism and insert the needle to the injection site, the second burst to pump the fluid out of the reservoir and into the living organism. Retraction of the needle is provided by the remainder of energy of the second energy source.

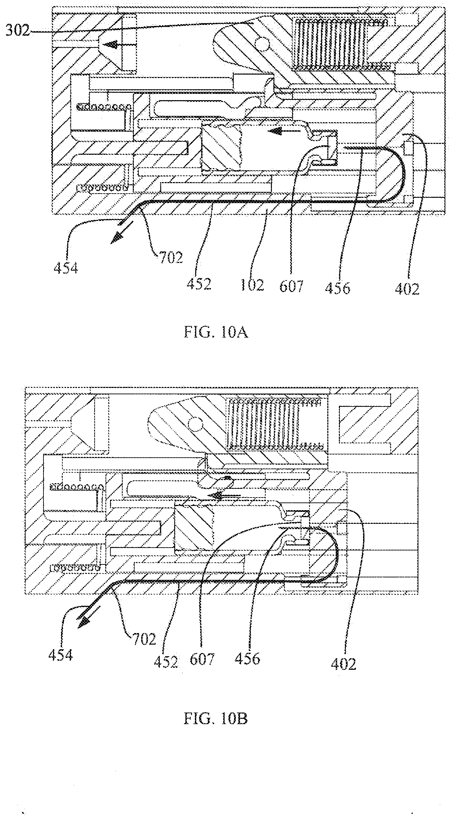

The following **FIGs. 9A to 9D** and **11 to 14B** show more details about the first embodiment of the mechanism of the device 1 according to the invention FIGs. **10A to 10B** and **12** show details of the second example, not belonging to the invention as defined in the claims but useful for the understanding thereof.

Referring now to **FIG. 9A****,** referring to step 6 of operation of the mechanism 100, the release of the insertion subassembly 300 by the trigger mechanism 200 is represented as the removal of a locking element 232 from a latch feature made up of an appropriate shape (latch 132 in the main structure 102) and another corresponding shape in the insertion subassembly structure 302 (latch 332 in the insertion subassembly).

Referring now to **FIGs. 9B** to **9D****,** referring to step 7 of operation of the mechanism 100, the insertion subassembly 300 starts moving as pushed by the insertion spring 352. Since the needle subassembly 400 is attached to the insertion subassembly 300, the needle subassembly 400 executes the same movement, bringing the extraction end 456 of the needle 452 to pierce the septum 604 (see in particular **FIG. 9D****)** and the injection end 454 of the needle 452 to pierce the skin of the living organism penetrating to the injection depth under the skin of the living organism.

Referring now to **FIGs. 10A** and **10B****,** in the second example, the needle extraction end 456 pierces the cartridge's septum 607 (as opposed to the reservoir's septum 604 in the above embodiment). In the example as described in the instant paragraph, the injection depth is not "under the skin" (as in the embodiment of the precedent paragraph), but "in or under the skin" of the living organism, such as in subcutaneous depth which may be at 1-2mm under the surface of the skin.

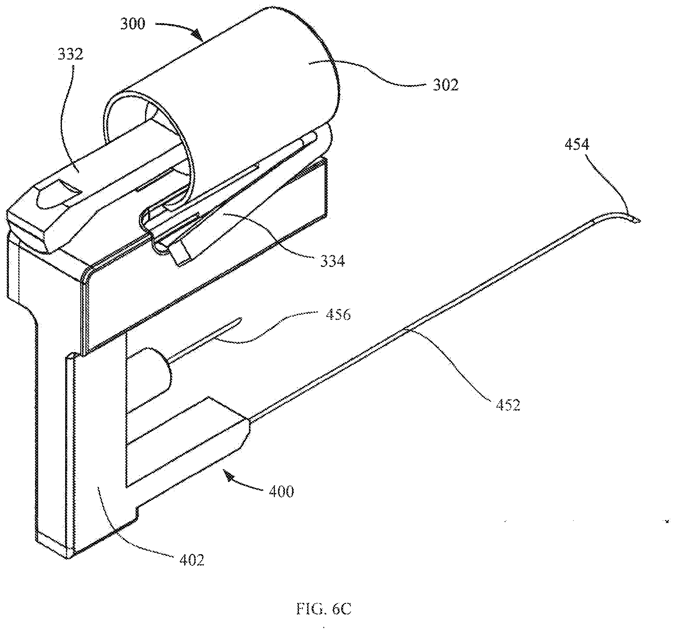

Referring now to **FIG. 11****,** in step 8 of operation of the mechanism 100, upon arrival, the needle subassembly structure 402 slides the intermediate part 512 of the dispensing assembly 500, opening the latch which is made up of feature 514 of the intermediate part 512, a latch feature 154 of the main structure 102 and a feature 504 of dispensing assembly structure 502. At this stage, the fluidic connection from the fluid container 602 through the needle extraction end 456 through the needle 452 through the needle injection end 454 is open, and the spring 552 of the dispensing subassembly is released. At the same time, the locking feature 334 (shown in **FIG. 6C****)** snaps in the corresponding feature 134 of the main structure 102, immobilizing the insertion subassembly 300 and the attached needle assembly 400 so that they can't move backwards.

Referring now to **FIG. 12****,** in the second example, the dispensing assembly structure 502 moves relative to the cartridge's container 603, pushing the stopper 605 inside the container 603, expelling the fluid 606 outside of the container 603 through the needle extraction end 456, through the needle 452, through the needle injection end 454 into the living organism.

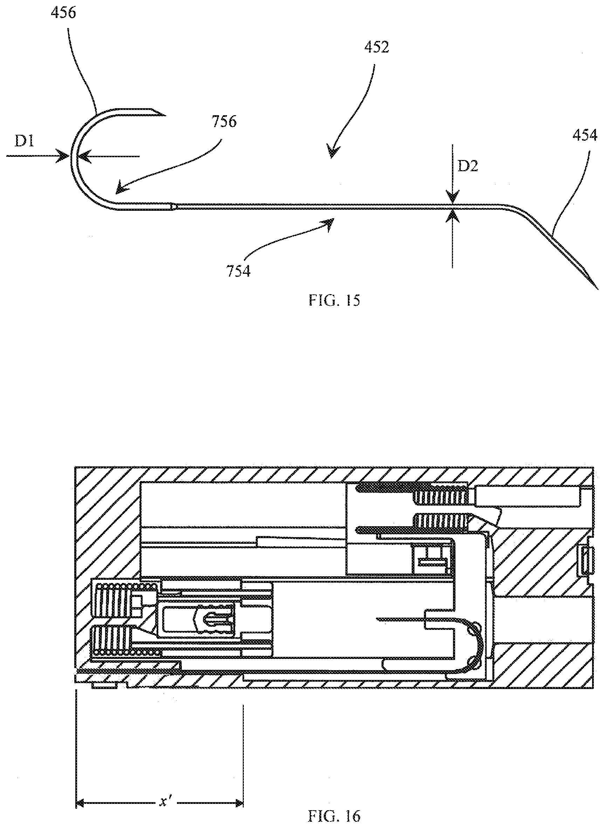

Note that dimension x (shown in **FIG. 12****)** or x' (shown in **FIG. 16****)** can be selected in the design phase of all embodiments shown herein, from 1mm or less to 40mm or more, depending on the application needs.

Referring now to **FIG. 13****,** in step 9 of operation of the mechanism 100, when released, the dispensing spring 552 pushes the reservoir container 602 against the needle subassembly structure 402, which generates a relative movement of the septum 604 towards the bottom of the container 602, pushing the fluid 606 out of the container 602 through the needle extraction end 456, through the needle 452, through the needle injection end 454 into the living organism.

Referring now to **FIGs. 14A** and **14B****,** in optional step 10 of operation of the mechanism 100, when the dispensing spring 552 which pushes the reservoir container 602 against the needle subassembly structure 402 reaches the point where the triggering feature 506 of the dispensing subassembly structure 502 pushes the release feature 336, the needle subassembly 400 is released from the insertion subassembly 300 and starts moving back to the position it had prior to the start of the operation. While moving backward, the needle subassembly 400, pushed by the dispensing subassembly 500, retracts the needle 452 until the needle injection end 454 is fully retracted within the main structure 102.

Note that the needle 452, optionally passes through a mandrel 702 that deforms the needle so as to prevent it from being reused. In one embodiment, the needle is deformed so as to be bowed, thus ensuring that buckling of the needle because it is no longer straight prevents it from passing through the mandrel once again.

Referring now to **FIG. 15****,** showing some further details of the needle 452. The first end of the needle, also referred to as needle extraction end 456 is shown in more detail. Further, the second end of the needle, also referred to as needle injection end 454 is shown in more detail. According to the invention, the needle 452 comprises an approx. 180° turn in the area of the first end of the needle. The diameter D1 of the needle in that area may be of a certain size, while the diameter D2 of the other portion of the needle may be larger, smaller or the same as D1. The portion with diameter D1 remains during operation inside the housing 2, while the portion of the needle with diameter D2 is configured to bend and at least partly inserted into the patient during operation. The portion of the needle with diameter D2 is on the side of needle's injection end 454. The needle 452 comprises an interior channel (not shown) going from one end to the other end of the needle. The diameter of the channel may be adapted to the fluid's viscosity. However, the energy of one or both energy sources may be adapted to the fluid's viscosity as well. In an even more preferred way, both the size of the diameter of the channel and the energy of one or both energy sources are adapted to the fluid's viscosity. In order to ensure deformation and buckling of the needle though the mandrel during operation, the diameter of the channel and the diameter D2 are selected so as to ensure proper deformation (bending and/or buckling). In a further embodiment, the needle may have varing diameter D2 to ensure proper bending and buckling. In another further embodiment, the channel may comprise a restrictor, such as a flow restrictor.

By placing and affixing or integrating into one housing one or more devices shown in FIGs. 5A-5B, 6A-6D, 7A-7B, 8A-8B, 9A-9D, 10A-10B, 11-13, 14A-14B, and 16 (such as adjacent or parallel to one another), the embodiments as described in the present description may be configured to accommodate more than one needle 454 in parallel (simultaneously, optionally side-by-side), such that the first ends 456 of these needles pierce the same septum or different septi for the same or different reservoirs. In a further alternative, the embodiments as described in the present description may be configured to handle more than one needle 454 in parallel, such that the first ends 456 of these needles pierce at least two septi, each septum being allocated to an individual reservoir. Using more than one needle simultaneously provides several advantages:
- it allows for using smaller needles which generate less pain when being inserted into the body of the living organism;
- it allows for simultaneously injecting several smaller doses of fluid into a living organism instead of one larger dose thereby generating less local tissue damage and therefore reducing the pain of the injection for the patient; and
- it allows for injecting the fluid at multiple locations thereby providing more efficient treatment of the patient, as the injected fluid will diffuse easier and faster within the body of the living organism.

Referring now to **FIG. 16****,** an alternative embodiment is shown, wherein the needle's injection end 454 is straight and remains straight during operation. For operation, the user applies the device body against the skin of the living organism such that the injection end of the needle 454 enters substantially orthogonal into the living organism. In this embodiment, the needle is not bent during operation. In addition, retraction of the needle may be accomplished by the user pulling the device away from the skin, as in a standard syringe.

Note that all mobile elements of the mechanism 100 subject to relative sliding or frictional forces may have a coating applied to their surfaces where appropriate to ensure low friction.

The fluid for injection is typically a fluid selected from a group of fluids consisting of peptides, proteins, hormones including insulin, calcitonin, calcitonin gene regulating protein, atrial natriuretic protein, colony stimulating factor, betaseron, erythropoietin (EPO), interferons such as .alpha., .beta. or gamma. interferon, somatropin, somatotropin, somastostatin, insulin-like growth factor (somatomedins), luteinizing hormone releasing hormone (LHRH), tissue plasminogen activator (TPA), growth hormone releasing hormone (GHRH), oxytocin, estradiol, growth hormones, leuprolide acetate, factor VIII, interleukins such as interleukin-2, and analogues or antagonists thereof, such as IL-1ra; analgesics such as fentanyl, sufentanil, butorphanol, buprenorphine, levorphanol, morphine, hydromorphone, hydrocodone, oxymorphone, methadone, lidocaine, bupivacaine, diclofenac, naproxen, paverin, and analogues thereof; anti-migraine agents such as sumatriptan, ergot alkaloids, and analogues thereof; anti-coagulant agents such as heparin, hirudin, and analogues thereof; anti-emetic agents such as scopolamine, ondansetron, domperidone, metoclopramide, and analogues thereof; cardiovacular agents, anti-hypertensive agents and vasodilators such as diltiazem, clonidine, nifedipine, verapamil, isosorbide-5-monotritate, organic nitrates, agents used in treatment of heart disorders, and analogues thereof; sedatives such as benzodiazepines, phenothiazines, and analogues thereof; chelating agents such as defroxanune, and analogues thereof; anti-diuretic agents such as desmopressin, vasopressin, and analogues thereof; anti-anginal agents such as fluorouracil, bleomycin, and analogues thereof; antineoplastics such as fluorouracil, bleomycin, and analogues thereof; prostaglandins and analogues thereof; and chemotherapy agents such as vincristine, and analogues thereof, treatments for attention deficit disorder, methylphenidate, fluvoxamine, bisoprolol, tacrolimus, sacrolimus and cyclosporin, vitamins suspended in a liquid carrier, antivenoms, syrums, medications, antibodies, Actemra (tocilizumab), Adcretris (brentuximab vedotin), Arzerra (ofatumumab), Avastin (bevacizumab), Benlysta (belimumab), Cimzia (certolizumab pegol), Erbitux (cetuximab), Herceptin (trastuzumab), Humira (adalimumab), Ilaris (canakinumab), Lucentis (ranibizumab), Mylotarg (gemtuzumab ozogamicin), Perjeta (pertuzumab), Prolia (denosumab), Remicade (infliximab), Simponi (golimumab), Soliris (eculizumab), Stelara (ustekinumab), Tysabri (natalizumab), Vectibix (panitumumab), Xgeva (denosumab), Xolair (omalizumab), Yervoy (ipilimumab), and Zevalin (ibritumomab tiuxetan).

**In** an advantage, the invention provides an easy to use, user friendly device that allows any person, even the patients themselves, to perform an injection of a fluid, of particular interest, a COVID-19 vaccine, without the intervention of medical personell, thereby reducing the burden on world healthcare systems.

It is another object of the invention to provide a user friendly device that allows a patient to inject themselveswithout requiring him to make an aggressive gesture like stabing himself.

In an advantage, the invention provides a device that can step up to the challenge of new molecular therapeutic innovations such as high viscosity fluids whose characteristics otherwise limit administration options.

In an advantage, the invention provides a device that can simultaneously inject several smaller doses of fluid into a living organism instead of one larger dose, generating less local tissue damage and therefore reducing the pain of the injection for the patient.

In an advantage, the invention provides a device with and extended product life cycle.

In an advantage, the invention provides a device that retracts the needle to prevent inadvertent harm that might be caused by an exposed needle.

In an advantage, the invention provides the option of injecting at an angle as the needle passes through a mandrel.

In an advantage, the invention helps prevent unsanitary or perhaps inadvertent reuse of the device, thus providing a safer means of injecting a fluid.

The device of the invention may be summarized as follows:
1. A fluid dispensing device 1 adapted for intramuscular and/or subcutaneous injection into a living organism, the device having an energy storage arrangement 352, 552 enclosed in a housing 2, the energy storage arrangement providing energy in at least two bursts, the first burst being that which is necessary to pierce the skin of the living organism and insert at least one needle 454 to the injection site, the second burst being that which is necessary to press the fluid out of the at least one reservoir 602 and into the living organism, and, optionally, to retract the at least one needle out of the living organism.
2. The device of feature set 1, wherein the first burst is provided by a first energy source 352 and the second burst is provided by a second energy source 552.
3. The device of any of the above feature sets, wherein the energy storage devices 352, 552 are springs.
4. The device of any of the above feature sets, wherein the at least one needle 452 has first and second ends which are adapted to pierce respective surfaces while translating together in a direction such that when used, the direction of movement of the first end of the at least one needle is parallel to a patient's skin and the direction of movement of the second end of the at least one needle is substantially non orthogonal to the patient's skin.
5. The device of any of the above feature sets, wherein the at least one needle passes through a mandrel which diverts the direction of translation of the at least one needle from one orientation, typically parallel, with respect to the patient's skin to a non-orthogonal angle with respect to the patient's skin.
6. The device of the above feature set wherein the mandrel 702 is adapted to deform the needle 452 as it passes therethrough.
7. The device of the above feature set, wherein the mandrel 702 is adapted to deform the needle beyond the needle's elastic limit thereby preventing re-use of the device.
8. The device of any of the above feature sets, wherein at least one thin, impermeable membrane 704 is adhesively attached to the housing 2 so as to cover and seal an at least one opening 706 in the device through which the at least one needle pierces and extends upon actuation for injection.
9. A method of operation of the device 1 includes the following steps:
   a. in case the device 1 is attached to a second device 1 as a pair, detaching the devices 1 to enable use one device at a time;
   b. removing a cover 4 unless a thin membrane 704, which the at least one needle 454 can pierce, is used to seal the device, in which case no cover is required;
   c. applying the device body 2 with pressure against the skin of the living organism;
   d. optionally increasing the pressure applied against the skin of the living organism until it is sufficient so that the optional detector 12 releases the push button 14 allowing manual triggering in the next step or alternatively, the detector 12 automatically actuating the trigger mechanism 200, in which latter case, skip to step f;
   e. pushing the push button 14, thereby actuating the trigger mechanism 200 which in turn releases the at least one insertion subassembly 300;
   f. pushing an at least one needle subassembly 400 using the insertion subassembly 300 until the needle injecting end 454 is inserted into the living organism at the injection depth, and the needle extraction end 456 has pierced an at least one septum 604 of the at least one reservoir 602;
   h. upon reaching this position, locking the insertion subassembly 300 to the main structure 102;
   i. locking the at least one needle subassembly 400, which is still attached to the at least one insertion subassembly 300, in place, while, at the same time, releasing the dispensing assembly 500, pushing the fluid reservoir subassembly 600 against the needle subassembly 400
   j. displacing the at least one septum 604 towards the bottom of the at least one fluid reservoir 602, acting as a piston and dispensing the fluid 606 through the needle extraction end 456, through the at least one needle 452, through the at least one needle injection end 454 into the living organism; and
   k. removing the device from the living organism.
10. The method of feature set 9, wherein just prior to removal in step k, when the dispensing subassembly 500 has emptied the at least one reservoir, the method includes the additional step of releasing the at least one needle subassembly 400 from the at least one insertion subassembly 300, which is then pushed back to its start position, thereby retracting the at least one needle 452 until the needle injection end 454 is fully retracted within the main structure 102 thereby preventing harm that might be caused by an exposed needle.
11. The method of operation of feature set 1, wherein the locking in step h is accomplished using a latch system (334).
12. The device of any of the above feature sets, wherein at least a portion (754) of the interior channel of the needle (452) conducting the fluid has a diameter different from that of another portion (756) of the interior channel of the needle.
13. The device of any of the above feature sets, wherein at least two needles within one housing are used simultaneously to conduct the fluid into the living organism.

Further, the invention should be considered as comprising all possible combinations of every feature described in the instant specification, appended feature sets, and/or drawing figures which may be considered new, inventive and industrially applicable.

It should be appreciated that the particular implementations shown and herein described are representative of the invention and its best mode and are not intended to limit the scope of the present invention in any way.

As will be appreciated by skilled artisans, the present invention may be embodied as a system or a device.

Moreover, the system contemplates the use, sale and/or distribution of any goods, services or information having similar functionality described herein.

The specification and figures should be considered in an illustrative manner. Accordingly, the scope of the invention should be determined by the appended claims rather than by merely the examples described above.

Benefits, other advantages and solutions mentioned herein are not to be construed as critical, required or essential features or components of any or all the claims.

As used herein, the terms "comprises", "comprising", or variations thereof, are intended to refer to a non-exclusive listing of elements, such that any apparatus, process, method, article, or composition of the invention that comprises a list of elements, that does not include only those elements recited. Unless otherwise explicitly stated, the use of the term "consisting" or "consisting of" or "consisting essentially of" is not intended to limit the scope of the invention to the enumerated elements named thereafter, unless otherwise indicated. Other combinations and/or modifications of the above-described elements, materials or structures used in the practice of the present invention may be varied or adapted by the skilled artisan to other designs.

### ADDENDUM

Following elements are disclosed in **FIGs. 1** to **3****:**
- 1: Dispensing device
- 2: Body of the dispensing device / housing
- 4: Cover
- 12: Detector of patient's skin presence (optional)/ pressure-sensitive safety release mechanism
- 14: Trigger button
- 16: Indicator device ready to use / used

Following elements are disclosed in **FIGs. 4** to **14B****:**
- 100: main mechanism
- 102: main structure
- 132: latch element
- 134: protrusion
- 154: latch element
- 200: trigger mechanism
- 232: latch element
- 300: insertion subassembly
- 302: insertion subassembly structure
- 332: latch element
- 334: latch element
- 336: latch release lever
- 352: insertion spring / energy source
- 400: needle subassembly
- 402: needle subassembly structure
- 452: needle
- 454: needle injection end
- 456: needle extraction end
- 500: dispensing subassembly

- 502: dispensing subassembly structure
- 504: latch element
- 506: protrusion
- 512: intermediate part
- 514: latch element
- 552: dispensing and retraction spring / energy source
- 600: reservoir subassembly
- 601: cartridge (standard cartridge or off the shelf cartridge)
- 602: reservoir body
- 604: septum
- 606: fluid
- 702: mandrel
- 704: impermeable membrane
- 706: opening
- 754: portion of the interior channel
- 756: portion of the interior channel

## Claims

1. A fluid dispensing device (1) adapted for intramuscular and/or subcutaneous, or intracutaneous, injection into a living organism, the device (1) having an energy storage arrangement (352, 552) enclosed in a main housing (2), the energy storage arrangement providing energy in at least two bursts, the first burst being that which is necessary to pierce the skin of the living organism and insert a needle (452) into the injection site, the second burst being that which is necessary to press the fluid out of at least one reservoir (602) and into the living organism, and, optionally, to retract the needle (452) out of the living organism, wherein the device (1) comprises:
- the main housing (2) enclosing the operative components of the device (1),
- the at least one fluid reservoir (602) located within the main housing (2),
- a needle subassembly (400) which comprises a structure (402) holding the needle (452), the needle (452) having a first end (456) and a second end (454) adapted for interfacing, on the first end, with a septum (604) disposed in the at least one reservoir (602) containing the fluid (606) to be dispensed, and at a second end (454) thereof, for inserting into a living organism, wherein when the septum (604) is pierced, the needle (452) is in fluid communication with the at least one fluid reservoir (602) and the device (1) expels the fluid (606) through the needle (452), through the second end (454) into the living organism, wherein the needle (452) comprises an approximately 180° turn in the area of the first end (456) of the needle (452), such that both needle ends point in the same direction for the first end to pierce the at least one reservoir and the second end to pierce the skin of the patient, and
- a trigger mechanism (200) configured to release an insertion subassembly (300) that pushes the needle subassembly (400), until the second end of the needle (454) is able to insert into the living organism to an injection depth, and the first end of the needle (456) has pierced the reservoir septum (604), the needle subassembly (400) being attached to the insertion subassembly (300) so that the needle subassembly (400) executes the same movement of the insertion subassembly (300) bringing the first end (456) of the needle (452) to pierce the septum (604), wherein the insertion subassembly (300) comprises a structure (302) containing an insertion spring (352), which is compressed prior to device (1) operation, the insertion subassembly (300) starts moving as pushed by the insertion spring (352),
- the device (1) further comprises a dispensing spring (552) pushing the at least one fluid reservoir (602) against the needle subassembly structure (402) to generate a relative movement of the septum (604) towards the bottom of the container (602), pushing the fluid (606) out of the at least one fluid reservoir (602) through the first end of the needle (456).

2. The device of the above claim, wherein the needle (452) has first and second ends (456, 454) which are adapted to pierce respective surfaces while translating together in a direction such that when used, the direction of movement of the first end of the needle (456) is parallel to a patient's skin and the direction of movement of the second end of the needle (454) is substantially non orthogonal to the patient's skin.

3. The device of any of the above claims wherein the needle (452) passes through a mandrel (702) which diverts the direction of translation of the needle (452) from one orientation, typically parallel, with respect to the patient's skin to a non-orthogonal angle with respect to the patient's skin.

4. The device of the above claim wherein the mandrel (702) is adapted to deform the needle (452) as it passes therethrough.

5. The device of the above claim, wherein the mandrel (702) is adapted to deform the needle (452) beyond the needle's elastic limit thereby preventing re-use of the device (1).

6. The device of any of the above claims, wherein at least one impermeable membrane (704) is adhesively attached to the housing (2) so as to cover and seal an at least one opening (706) in the device (1) through which the needle (452) pierces and extends upon actuation for injection.

7. The device of any of the above claims, wherein the needle (452) comprises an interior channel going from one end (454, 456) to the other end (456, 454) of the needle (452), at least a portion (754) of the interior channel of the needle (452) conducting the fluid (606) has a diameter (D2) different from the diameter (D1) of another portion (756) of the interior channel of the needle (452).

8. The device of any of the above claims, wherein at least two needles (452) within the housing (2) are used simultaneously to conduct the fluid (606) into the living organism.

9. The device of any of the above claims, wherein the structure (302) of the insertion subassembly (300) comprises a release feature (336) for releasing the needle subassembly (400) from the insertion subassembly (300) so that needle subassembly (400) can return to the initial position and the needle (452) retracted.

10. The device of any of the above claims, further comprising a pressure-sensitive safety release mechanism (12) configured to release a push button (14) only when the pressure applied on the skin of the living organism is above a predefined threshold to ensure correct disposition/retention for optimal operation of the device (1) prior to allowing the push button (14) to be pressed for triggering the actuation of the trigger mechanism (200).

11. The device of any of the above claims, further comprising an indicator (16) for allowing the user to check whether the device (1) has been used or is available for use.

12. An assembly comprising a pair of fluid dispensing devices (1) according to any one of the preceding claims attached together.

## Patentansprüche

1. Fluidabgabevorrichtung (1), die zur intramuskulären und/oder subkutanen oder intrakutanen Injektion in einen lebenden Organismus angepasst ist, wobei die Vorrichtung (1) eine Energiespeicheranordnung (352, 552) aufweist, die in einem Hauptgehäuse (2) eingeschlossen ist, wobei die Energiespeicheranordnung Energie in mindestens zwei Schüben bereitstellt, wobei der erste Schub derjenige ist, der erforderlich ist, um die Haut des lebenden Organismus zu durchstechen und eine Nadel (452) in die Injektionsstelle einzuführen, der zweite Schub derjenige ist, der erforderlich ist, um das Fluid aus mindestens einem Reservoir (602) in den lebenden Organismus zu drücken und gegebenenfalls die Nadel (452) aus dem lebenden Organismus zurückzuziehen, wobei die Vorrichtung (1) umfasst:
- das Hauptgehäuse (2), das die operativen Komponenten der Vorrichtung (1) umschließt,
- das mindestens eine Fluidreservoir (602), das sich innerhalb des Hauptgehäuses (2) befindet,
- eine Nadelunterbaugruppe (400), die eine Struktur (402) umfasst, welche die Nadel (452) hält, wobei die Nadel (452) ein erstes Ende (456) und ein zweites Ende (454) aufweist, die so beschaffen sind, dass sie an dem ersten Ende mit einem Septum (604) zusammenwirken, das in dem mindestens einen Behälter (602) angeordnet ist, der das abzugebende Fluid (606) enthält, und an ihrem zweiten Ende (454) in einen lebenden Organismus eingeführt werden, wobei, wenn das Septum (604) durchstochen wird, die Nadel (452) in Fluidverbindung mit dem mindestens einen Fluidreservoir (602) steht und die Vorrichtung (1) das Fluid (606) durch die Nadel (452) durch das zweite Ende (454) in den lebenden Organismus ausstößt, wobei die Nadel (452) eine ungefähr 180°-Drehung im Bereich des ersten Endes (456) der Nadel (452) umfasst, sodass beide Enden der Nadeln in die gleiche Richtung zeigen, damit das erste Ende das mindestens eine Reservoir durchsticht und das zweite Ende die Haut des Patienten durchsticht, und
- einen Auslösemechanismus (200), der konfiguriert ist, um eine Einführungsunterbaugruppe (300) freizugeben, welche die Nadelunterbaugruppe (400) schiebt, bis das zweite Ende der Nadel (454) in der Lage ist, in den lebenden Organismus bis zu einer Injektionstiefe einzuführen, und das erste Ende der Nadel (456) das Reservoirseptum (604) durchstochen hat, die Nadelunterbaugruppe (400) an der Einführungsunterbaugruppe (300) befestigt ist, sodass die Nadelunterbaugruppe (400) die gleiche Bewegung der Einführungsunterbaugruppe (300) ausführt, die das erste Ende (456) der Nadel (452) veranlasst, das Septum (604) zu durchstechen, wobei die Einführungsunterbaugruppe (300) eine Struktur (302) umfasst, die eine Einführungsfeder (352) enthält, die vor der Bedienung der Vorrichtung (1) zusammengedrückt wird, wobei die Einführungsunterbaugruppe (300) sich zu bewegen beginnt, wenn sie von der Einführungsfeder (352) gedrückt wird,
- die Vorrichtung (1) ferner eine Abgabefeder (552) umfasst, die das mindestens eine Fluidreservoir (602) gegen die Struktur der Nadelunterbaugruppe (402) drückt, um eine relative Bewegung des Septums (604) in Richtung des Bodens des Behälters (602) zu erzeugen, wodurch das Fluid (606) aus dem mindestens einen Fluidreservoir (602) durch das erste Ende der Nadel (456) herausgedrückt wird.

2. Vorrichtung nach dem vorstehenden Anspruch, wobei die Nadel (452) ein erstes und ein zweites Ende (456, 454) hat, die so beschaffen sind, dass sie jeweilige Oberflächen durchstechen, während sie sich gemeinsam in einer solchen Richtung verschieben, dass bei der Verwendung die Bewegungsrichtung des ersten Endes der Nadel (456) parallel zur Haut eines Patienten verläuft und die Bewegungsrichtung des zweiten Endes der Nadel (454) im Wesentlichen nicht orthogonal zur Haut des Patienten verläuft.

3. Vorrichtung nach einem der vorstehenden Ansprüche, wobei die Nadel (452) durch einen Dorn (702) geführt wird, der die Bewegungsrichtung der Nadel (452) von einer Ausrichtung, in der Regel parallel, in Bezug auf die Haut des Patienten in einen nicht orthogonalen Winkel in Bezug auf die Haut des Patienten umlenkt.

4. Vorrichtung nach dem vorstehenden Anspruch, wobei der Dorn (702) angepasst ist, um die Nadel (452) beim Durchgang durch ihn zu verformen.

5. Vorrichtung nach dem vorstehenden Anspruch, wobei der Dorn (702) angepasst ist, um die Nadel (452) über ihre Elastizitätsgrenze hinaus zu verformen, wodurch eine Wiederverwendung der Vorrichtung (1) verhindert wird.

6. Vorrichtung nach einem der vorstehenden Ansprüche, wobei mindestens eine undurchlässige Membran (704) klebend an dem Gehäuse (2) befestigt ist, um somit mindestens eine Öffnung (706) in der Vorrichtung (1) abzudecken und abzudichten, durch welche die Nadel (452) bei Betätigung zur Injektion eindringt und sich erstreckt.

7. Vorrichtung nach einem der vorstehenden Ansprüche, wobei die Nadel (452) einen Innenkanal umfasst, der von einem Ende (454, 456) zum anderen Ende (456, 454) der Nadel (452) verläuft mindestens ein Abschnitt (754) des Innenkanals der Nadel (452), der das Fluid (606) führt, einen Durchmesser (D2) hat, der sich vom Durchmesser (D1) eines anderen Abschnitts (756) des Innenkanals der Nadel (452) unterscheidet.

8. Vorrichtung nach einem der vorstehenden Ansprüche, wobei mindestens zwei Nadeln (452) innerhalb des Gehäuses (2) gleichzeitig verwendet werden, um das Fluid (606) in den lebenden Organismus zu führen.

9. Vorrichtung nach einem der vorstehenden Ansprüche, wobei die Struktur (302) der Einführungsunterbaugruppe (300) ein Freigabemerkmal (336) zum Freigeben der Nadelunterbaugruppe (400) von der Einführungsunterbaugruppe (300) umfasst, sodass die Nadelunterbaugruppe (400) in die Ausgangsposition zurückkehren kann und die Nadel (452) zurückgezogen werden kann.

10. Vorrichtung nach einem der vorstehenden Ansprüche, ferner umfassend einen druckempfindlichen Sicherheitsauslösemechanismus (12), der konfiguriert ist, um einen Druckknopf (14) nur dann freizugeben, wenn der auf die Haut des lebenden Organismus ausgeübte Druck über einem vordefinierten Schwellenwert liegt, um eine korrekte Disposition/Haltung für einen optimalen Betrieb der Vorrichtung (1) zu gewährleisten, bevor der Druckknopf (14) zum Auslösen der Betätigung des Auslösemechanismus (200) gedrückt werden kann.

11. Vorrichtung nach einem der vorstehenden Ansprüche, ferner umfassend eine Anzeige (16), die es dem Benutzer ermöglicht, zu überprüfen, ob die Vorrichtung (1) benutzt wurde oder zur Benutzung verfügbar ist.

12. Baugruppe, umfassend ein Paar von Fluidabgabevorrichtungen (1) nach einem der vorstehenden Ansprüche, die aneinander befestigt sind.

## Revendications

1. Dispositif de distribution de fluide (1) adapté à l'injection intramusculaire et/ou sous-cutanée ou intracutanée dans un organisme vivant, le dispositif (1) ayant un agencement de stockage d'énergie (352, 552) enfermé dans un boîtier principal (2), l'agencement de stockage d'énergie fournissant de l'énergie en au moins deux rafales, la première étant celle qui est nécessaire pour percer la peau de l'organisme vivant et insérer une aiguille (452) dans le site d'injection, la seconde rafale étant celle qui est nécessaire pour presser le fluide hors d'au moins un réservoir (602) et dans l'organisme vivant, et, facultativement, pour rétracter l'aiguille (452) hors de l'organisme vivant, dans lequel le dispositif (1) comprend :
- le boîtier principal (2) renfermant les composants opérationnels du dispositif (1),
- l'au moins un réservoir de fluide (602) situé à l'intérieur du boîtier principal (2),
- un sous-ensemble d'aiguille (400) qui comprend une structure (402) contenant l'aiguille (452), l'aiguille (452) ayant une première extrémité (456) et une seconde extrémité (454) adaptées pour réaliser une interface, sur la première extrémité, avec un septum (604) disposé dans l'au moins un réservoir (602) contenant le fluide (606) à distribuer, et au niveau d'une seconde extrémité (454) de celui-ci, pour une insertion dans un organisme vivant, dans lequel, lorsque le septum (604) est percé, l'aiguille (452) est en communication fluidique avec l'au moins un réservoir de fluide (602) et le dispositif (1) expulse le fluide (606) à travers l'aiguille (452), par la seconde extrémité (454) dans l'organisme vivant, dans lequel l'aiguille (452) comprend un virage d'environ 180° dans la zone de la première extrémité (456) de l'aiguille (452), de telle sorte que les deux extrémités de l'aiguille pointent dans la même direction afin que la première extrémité perce l'au moins un réservoir et que la seconde extrémité perce la peau du patient, et
- un mécanisme de déclenchement (200) configuré pour libérer un sous-ensemble d'insertion (300) qui pousse le sous-ensemble d'aiguille (400), jusqu'à ce que la seconde extrémité de l'aiguille (454) puisse s'insérer dans l'organisme vivant à une profondeur d'injection, et que la première extrémité de l'aiguille (456) ait percé le septum de réservoir (604), le sous-ensemble d'aiguille (400) étant fixé au sous-ensemble d'insertion (300) de sorte que le sous-ensemble d'aiguille (400) exécute le même mouvement que le sous-ensemble d'insertion (300) amenant la première extrémité (456) de l'aiguille (452) à percer le septum (604), dans lequel le sous-ensemble d'insertion (300) comprend une structure (302) contenant un ressort d'insertion (352), qui est comprimé avant le fonctionnement du dispositif (1), le sous-ensemble d'insertion (300) commence à se déplacer lorsqu'il est poussé par le ressort d'insertion (352),
- le dispositif (1) comprend en outre un ressort de distribution (552) poussant l'au moins un réservoir de fluide (602) contre la structure de sous-ensemble d'aiguille (402) pour générer un mouvement relatif du septum (604) vers le fond du récipient (602), poussant le fluide (606) hors de l'au moins un réservoir de fluide (602) à travers la première extrémité de l'aiguille (456).

2. Dispositif selon la revendication précédente, dans lequel l'aiguille (452) a des première et seconde extrémités (456, 454) qui sont adaptées pour percer des surfaces respectives tout en effectuant une translation ensemble dans une direction de telle sorte que, lors de l'utilisation, la direction de mouvement de la première extrémité de l'aiguille (456) est parallèle à la peau d'un patient et la direction de mouvement de la seconde extrémité de l'aiguille (454) est sensiblement non orthogonale à la peau du patient.

3. Dispositif selon l'une quelconque des revendications précédentes, dans lequel l'aiguille (452) passe à travers un mandrin (702) qui dévie la direction de translation de l'aiguille (452) d'une orientation, typiquement parallèle, par rapport à la peau du patient à un angle non orthogonal par rapport à la peau du patient.

4. Dispositif selon la revendication précédente, dans lequel le mandrin (702) est adapté pour déformer l'aiguille (452) lors de son passage.

5. Dispositif selon la revendication précédente, dans lequel le mandrin (702) est adapté pour déformer l'aiguille (452) au-delà de la limite élastique de l'aiguille, empêchant de ce fait la réutilisation du dispositif (1).

6. Dispositif selon l'une quelconque des revendications précédentes, dans lequel au moins une membrane imperméable (704) est fixée par adhésif au boîtier (2) de manière à couvrir et à sceller au moins une ouverture (706) dans le dispositif (1) à travers laquelle l'aiguille (452) perce et s'étend lors de l'actionnement pour l'injection.

7. Dispositif selon l'une quelconque des revendications précédentes, dans lequel l'aiguille (452) comprend un canal intérieur allant d'une extrémité (454, 456) à l'autre extrémité (456, 454) de l'aiguille (452), au moins une partie (754) du canal intérieur de l'aiguille (452) conduisant le fluide (606) a un diamètre (D2) différent du diamètre (D1) d'une autre partie (756) du canal intérieur de l'aiguille (452).

8. Dispositif selon l'une quelconque des revendications précédentes, dans lequel au moins deux aiguilles (452) à l'intérieur du boîtier (2) sont utilisées simultanément pour conduire le fluide (606) dans l'organisme vivant.

9. Dispositif selon l'une quelconque des revendications précédentes, dans lequel la structure (302) du sous-ensemble d'insertion (300) comprend un élément de libération (336) permettant de libérer le sous-ensemble d'aiguille (400) du sous-ensemble d'insertion (300) de sorte que le sous-ensemble d'aiguille (400) peut revenir à la position initiale et que l'aiguille (452) est rétractée.

10. Dispositif selon l'une quelconque des revendications précédentes, comprenant en outre un mécanisme de libération de sécurité sensible à la pression (12) conçu pour libérer un bouton-poussoir (14) uniquement lorsque la pression appliquée sur la peau de l'organisme vivant est au-dessus d'un seuil prédéfini afin d'assurer une disposition/rétention correcte pour un fonctionnement optimal du dispositif (1) avant de permettre d'appuyer sur le bouton-poussoir (14) pour déclencher l'actionnement du mécanisme de déclenchement (200).

11. Dispositif selon l'une quelconque des revendications précédentes, comprenant en outre un indicateur (16) permettant à l'utilisateur de vérifier si le dispositif (1) a été utilisé ou s'il est disponible pour une utilisation.

12. Ensemble comprenant une paire de dispositifs de distribution de fluide (1) selon l'une quelconque des revendications précédentes attachés l'un à l'autre.
